# EUROPEAN PATENT APPLICATION

(11) **EP 2 719 355 A2**
(43) Date of publication of application: **16.04.2014**
(21) Application number: 13187445.5
(22) Date of filing: 04.10.2013
(51) Int. Cl.: A61B 19/00

(54) **Clutched-gear handle for fiducial deployment**

(30) Priority: 11.10.2012 US 201261712611 P
(71) Applicant: Cook Medical Technologies LLC, Bloomington, IN 47404 (US)
(72) Inventor: Clancy, Michael, Monaleen Co. Limerick (IE); McGrath, Darach, Nenagh Co. Tipperary (IE); Toomey, Ciaran, Rathcormac Co. Cork (IE); Campbell, Triona, Killaloe Co. Clare (IE); Mulcahy, Patrick, Nenagh Co. Tipperary (IE); Keady, Fionan, Glenamaddy Co. Galaway (IE)
(74) Representative: Garratt, Peter Douglas

(57) **Abstract**

A clutched-gear handle (1650) for a fiducial (400) deployment system including an advancement mechanism comprising: a handle (1654), a stylet (1660) and an actuation mechanism (1670); said actuation mechanism (1670) including at least one rotatable gear (1664,1666) mechanically communicating between an actuation member, embodied as a lever, a knob, or a button, and at least one rack (1676) attached to the stylet (1660), where the mechanism is constructed such that actuating the actuation mechanism (1670) rotates the at least one unidirectionally rotatable gear and thereby communicates mechanical force through the at least one rack (1676) to the stylet (1660) in a manner that distally advances at least one of the plurality of fiducials (400) past the detent and distally out of the needle lumen.

## Description

### CROSS-REFERENCE TO RELATED APPLICATIONS

This application claims priority to U.S. provisional application Serial No. 61/712,611, filed October 11, 2012, which is incorporated by reference herein in its entirety.

### TECHNICAL FIELD

Embodiments disclosed herein relate generally to a medical device system including one or more fiducials and methods of use for same. More particularly, the disclosed embodiments pertain to handle mechanisms and systems including same for deploying fiducials, and methods of use for same.

### BACKGROUND

Medical procedures often require locating and treating target areas within a patient. Focused, dose-delivery radiation therapy requires locating the target with a high degree of precision to limit damaging healthy tissue around the target. It is particularly important to know or estimate the precise location of the target in radiation oncology because it is desirable to limit the exposure of adjacent body parts to the radiation in a patient already suffering the depredations of cancer. However, in all treatment procedures, whether radiologic or otherwise, it is most desirable to be able to accurately target a region to be treated.

In many applications, it is not possible to directly view a treatment target or portion thereof (such as, for example, a cancerous tumor, cyst, pseudocyst, or other target) that needs to be acted on in some manner. As one example, when treating a lung or pancreatic tumor with radiation, it may not possible to view the actual tumor within the patient immediately before the radiation treatment. It is therefore highly advantageous to have some mechanism for permitting the tumor to be located accurately so that the radiation treatment can be targeted at the tumor while avoiding damage to healthy tissue.

Even for target regions that may be visualized using CAT (computer-assisted tomography) scans, MRI (magnetic resonance imaging), x-rays, ultrasound, or other techniques, difficulties often arise in targeting a treatment. This is particularly true for target regions within a torso of a patient and soft tissue regions. Due to the mobility of tissues in those regions (e.g., movement of internal organs during respiration and/or digestion, the movement of breast tissue with any change of body position, etc.), a target region may not remain fixed relative to anatomical landmarks and/or to marks that can be placed onto an external surface of a patient's body during one of those visualization procedures.

Several techniques have been developed to address this problem. One such technique is to place markers into the patient along the margins of the target region. The markers may be active (e.g., emitting some kind of signal useful in targeting a therapy) or passive (e.g., non-ferromagnetic metallic markers - called fiducials - that can be used for targeting under ultrasound, MRI, x-ray, or other targeting techniques, which may be included in a treatment device).

A fiducial is typically formed of a radio-opaque material that the target can be effectively located and treated with a device that targets a site using the fiducials as positional markers under radiographic detection. Typically, the fiducials may be inserted into the patient during a simple operation. Percutaneous placement is most commonly used. However, use of minimally-invasive placement via an endoscope has recently developed for fiducial placement into a patient's internal organs. For example, percutaneous placement of fiducials along the margins of a pancreatic tumor can be complex and painful (particularly for obese patients, where the needle size is necessarily larger). Another process using percutaneously implanted objects in a patient is brachytherapy. In brachytherapy, radioactive sources or "seeds" are implanted into and/or adjacent a tumor to provide a high dose of radiation to the tumor, but not the healthy tissue surrounding the tumor.

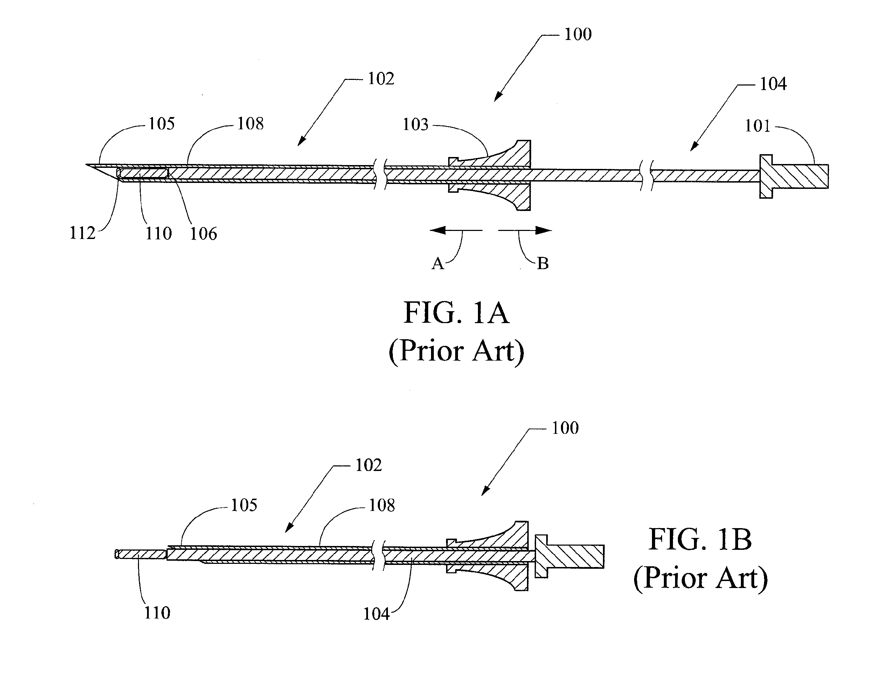

FIGS. 1A and 1B show longitudinal sectional views of a two-piece introducer 100 of the prior art useful for placement of brachytherapy seeds or fiducials. Referring first to FIG. 1A, the introducer 100 includes a needle 102 and a stylet 104 slidably disposed within the needle 102. The stylet 104 includes a first handle 101 and a blunt distal end 106. The needle 102 includes a second handle 103 and a bevel-tipped cannula 108 extending through the second handle 103. The cannula 108 is configured to hold a seed/fiducial 110. The cannula 108 has a distal tip 105 configured for percutaneous implantation of the seed/fiducial 110 into the patient.

In a "pre-loaded configuration," the seed/fiducial 110 is retained in the cannula 108 by a plug 112 made from bone wax or other suitable bio-compatible material(s). This is typically accomplished by a "muzzle-loading" technique where the fiducial is placed into the distal needle and then held in place by the bone wax plug. This can present some challenges, as the bone wax plug 112 can be visible as an artifact in the patient, potentially interfering with clear visualization of body structures or treatment devices. With this configuration, the cannula 108 must be withdrawn and reloaded after delivery of each seed/fiducial 110. If the target locations for the fiducials are very far apart, use of a single percutaneous introducer cannula/trocar for multiple introductions of the cannula 108 may not be possible. In such a circumstance, the patient must endure several percutaneous punctures (and the increased attendant risk of infection for each).

To implant the desired arrangement of seeds/fiducials 110 at a target location in a patient, an operator pushes the cannula 108 in a first direction (arrow A) to insert the tip 105 into the patient (typically under fluoroscopic visualization). The operator then pushes the second handle 103 further in the first direction to position the tip 105 at the desired depth within the patient where a seed/fiducial 110 is to be implanted. Throughout this motion, the operator moves the needle 102 and the stylet 104 together as a unit. At the desired depth/location, the operator grasps the first handle 101 with one hand and the second handle 103 with the other hand. Then, the operator holds the first handle 101 stationary while simultaneously sliding the second handle 103 back in a second direction (arrow B) toward the first handle 101. As shown in FIG. 1B, this movement causes the cannula 108 to retract over the seed/fiducial 110 to implant it in the patient. Alternatively, the operator may move the first handle 101 in the first direction (arrow A) while sliding the second handle 103 back in the second direction (arrow B). This causes the stylet 104 to push the seeds 110 out of the cannula 108. The procedure is then repeated to place other seeds/fiducials 110. When being used for targeting of radiation therapy, a minimum of three fiducials is typically required.

As will be appreciated from the disclosed structure, after deploying one fiducial, one may alternatively reload the introducer 100 from the proximal end by completely withdrawing the stylet 104, then placing another fiducial into the needle lumen and advancing it therethrough to a second location to which the distal needle tip 105 has been directed (a "breech-loading" technique). Provided that the fiducial target sites are sufficiently close together to allow this technique, it can reduce the number of percutaneous punctures or other access procedures needed to place more than one fiducial. However, it creates a problem for procedures where ultrasound is being used or is to be used in the near-future because it introduces air pockets into the tissue and related fluids. Those air pockets with tissue and/or fluid are echogenic in a manner that can interfere with ultrasound visualization of a target area and/or tools being used to diagnose or treat in/around the area. In some brachytherapy techniques, a series of fiducials may be preloaded into the needle - either separately or connected by a suture or similar device - then placed together in fairly close proximity; however, such a technique typically is not effective for placing three or more fiducials in sufficiently disparate locations to use for targeting a treatment relative to, for example, margins of a tumor. This may also be true for multifiducial systems that rely upon a distal plug to retain fiducials, which are thereafter released freely, in contrast with systems according to the present invention, which are configured for controlled serial release (e.g., one at a time, two at a time, or some other user-controlled retention and release of a pre-determined number of fiducials).

The process is similar when implemented endoscopically in the manner developed rather recently, except that the needle and stylet are of the type known in the art for use through the working channel of an endoscope. One limitation of current endoscopic techniques is the size of fiducial that can be introduced. With the size limitation of endoscope working channels, the largest needle that can typically be used without risking bending, crimping, curving or otherwise damaging a needle (that does not have an internal stylet or other support) during advancement out of the endoscope to an anatomical target is a 19-gauge needle. This limits the size of the fiducial that can be introduced through the needle lumen using current, cylindrical fiducials. The endoscopic technique generally suffers from the same reloading problems as described above. Even though the external percutaneous punctures are not an issue, having to withdraw and reload takes up valuable time and complicates the procedure, potentially requiring additional personnel, whether only the stylet is withdrawn for "breech-loading" or the entire device is withdrawn for "muzzle-loading."

It would be desirable to use ultrasound, and particularly endoscopic ultrasound (EUS) for navigation and placement of fiducials. As such it would be desirable to provide and use the largest possible fiducial that will provide improved echogenicity based on its size and echogenic profile. It would be desirable to provide multiple fiducials in a needle that can be introduced in a controlled serial manner (one, or some other predetermined number, at a time) rather than requiring manual reloading after placement of each fiducial.

### BRIEF SUMMARY

Embodiments of a fiducial deployment system described herein may include one or more of: one or a plurality of fiducials having one or more protuberances, a slotted needle configured for delivering a plurality of fiducials in serial fashion where the slot receives the fiducial protuberances without a detent that occupies any internal diameter needle lumen portion, a handle configured for controlling the serial delivery by user-operated deployment of a predetermined number of fiducials, and a method of delivering fiducials in a target region.

There is disclosed a clutched-gear handle for a fiducial deployment system including an advancement mechanism comprising:
an actuation member and an elongate first handle member, including a central longitudinal axis defined by an elongate outer body of the first handle member, the outer body including a body lumen longitudinally disposed therethrough;
where a stylet extends through at least a portion of the first handle member along or generally aligned with the central longitudinal axis;
an axle mounted rotatably in the first handle member, disposed adjacent to and transverse of the central longitudinal axis thereof;
a clutched gear mounted rotatably to the axle by a one-way clutch bearing allowing unidirectional rotation of the clutched gear around the axle, and a drive gear fixed to the axle;
where the actuation member is disposed in reciprocating mechanical communication with one of the drive gear or the clutched gear; and
a rack member engaged with and generally longitudinally aligned with the stylet, where a surface of the rack member mechanically communicates with a surface of the other of the drive gear or the clutched gear such that actuation of the actuation member is mechanically translated to unidirectional movement of the stylet via the rack and gears.

There is also disclosed a clutched-gear handle for a fiducial deployment system including an advancement mechanism comprising: a handle; a stylet and an actuation mechanism, said actuation mechanism including at least one rotatable gear mechanically communicating between an actuation member, embodied as a lever, a knob, or a button, and at least one rack attached to the stylet, where the mechanism is constructed such that actuating the actuation mechanism rotates the at least one unidirectionally rotatable gear and thereby communicates mechanical force through the at least one rack to the stylet in a manner that distally advances at least one of the plurality of fiducials past the detent and distally out of the needle lumen.

### BRIEF DESCRIPTION OF THE DRAWINGS

FIGS. 1A-1B show a prior art fiducial introducer and method of use;

FIGS. 2A-2C show an embodiment of a fiducial from, respectively, top, side, and transverse section views;

FIG. 3 shows a top view of a slotted needle embodiment;

FIG. 3A shows a top view of another slotted needle embodiment;

FIGS. 4-4B show, respectively, a top perspective view, a longitudinal section view, and a transverse section view of a distal fiducial deployment system portion;

FIGS. 5A-5C show a method of placing fiducials;

FIGS. 6A-6B show a handle embodiment for a fiducial deployment system;

FIGS. 7A-7C show, respectively, a cutaway view, an assembled view, and an alternative cutaway view with distal needle of an advancement mechanism embodiment for a fiducial deployment system;

FIGS. 8A-8B show, respectively, a cutaway view and an assembled view with distal needle of an advancement mechanism embodiment for a fiducial deployment system; and

FIG. 9 shows another handle embodiment for a fiducial deployment system.

### DETAILED DESCRIPTION

The terms "proximal" and "distal" are used herein in the common usage sense where they refer respectively to a handle/doctor-end of a device or related object and a tool/patient-end of a device or related object.

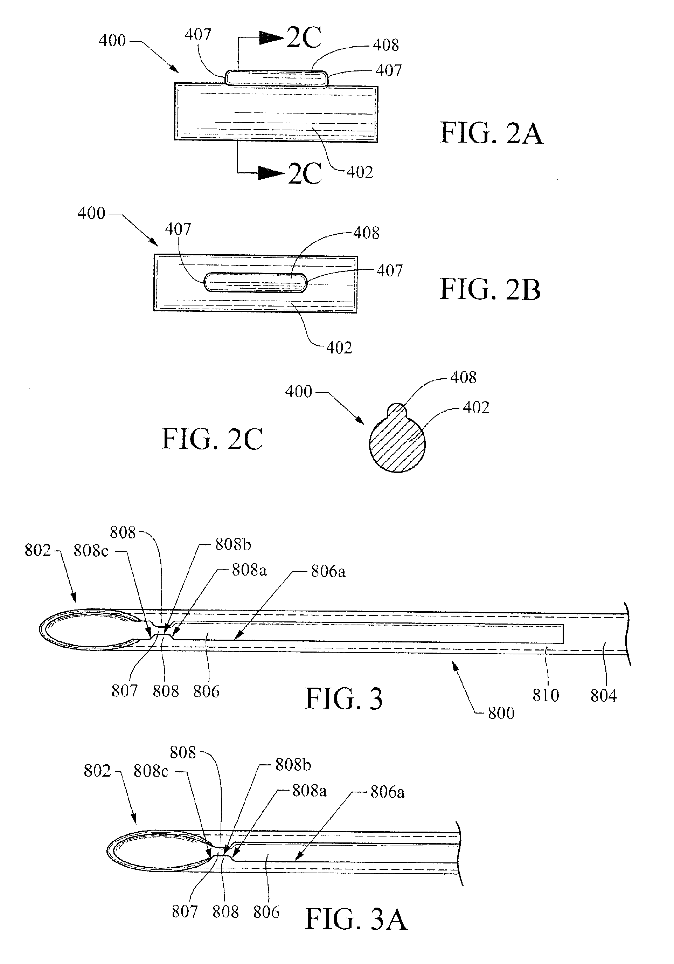

A variety of fiducial and needle configurations may be used in keeping with the present embodiments including those described in U.S. Pat. App. Publ. Nos. 2010/0280367 and 2011/0152611 to Ducharme et al., U.S. Pat. App. Ser. Nos. 13/526,991 to McHugo et al., 13/526,008 to Lavelle et al., and 13/645,614 to Murray et al., each of which is incorporated by reference herein in its entirety. One embodiment, illustrated with reference to FIGS. 2A-2C, of a fiducial 400 has a generally columnar body that is generally cylindrical with a generally circular transverse cross-section. A longitudinal surface face of the body may be dimpled to enhance its ability to reflect ultrasound waves and thereby provide a desirable echogenic profile. This dimpled characteristic may alternatively be embodied as a different irregular, patterned, or textured surface feature (e.g., knurled, ribbed) that may enhance the echogenicity of the fiducial 400, which will aid in visualizing it during EUS-guided placement, and allow it to be used in ultrasound visualization of a target site being marked by one or more fiducials 400 (e.g., a tumor).

Such a fiducial 400 preferably will be formed of a radio-opaque, non-ferromagnetic material such as, for example, gold, platinum, palladium, iridium, or alloys thereof, with one preferred embodiment including an alloy of palladium with rhenium (advantages of which may include desirable radio-opacity, market-price stability superior to gold, and ultrasound-reflectivity/echogenicity due to density). Being radio-opaque will allow the fiducial to be used in deployment techniques using fluoroscopy, as well as making it detectible/ visualizable by radiographic means during a treatment or other procedure where it may be desirable to know the location(s) of one or more fiducials. Being non-ferromagnetic will lessen the likelihood that visualization techniques or other procedures employing magnetic fields such as, for example, MRI, will re-orient or otherwise dislodge a fiducial. Echogenic construction of a fiducial or needle may be enhanced by surface texture, but can also be provided by structural inclusions such as embedded bubbles or beads that provide for a different ultrasound reflectivity than material surrounding them. Fiducials may also be coated with a material (e.g., parylene) configured to reduce backscatter during radiography.

In a preferred embodiment, the fiducial 400 is configured and dimensioned for passage through and release from a needle lumen. For an endoscopic delivery system, the fiducial body 402 (exclusive of the protuberance) preferably will have an outer diameter (OD) of about the same or less than the inner diameter (ID) of a needle lumen, but the OD of the fiducial body preferably will be no greater than the needle ID. As used herein, the OD of the fiducial refers to an imaginary circle (or other geometric shape) whose outermost boundaries all fit within the ID of the needle lumen. In other words, it is preferable that the fiducial is dimensioned to fit slidably into the needle lumen, except the protuberance, which projects into the slot.

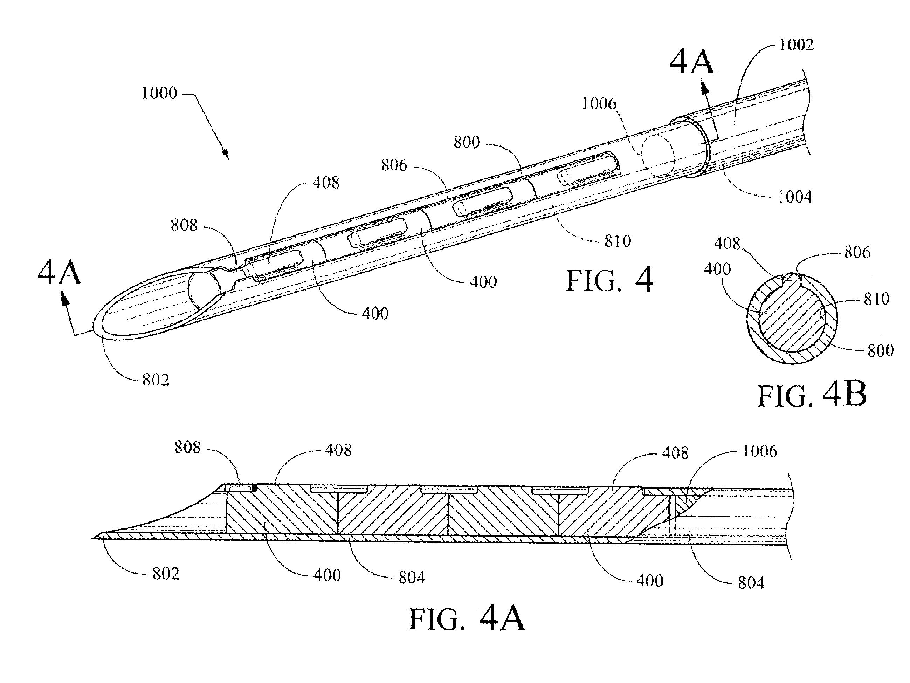

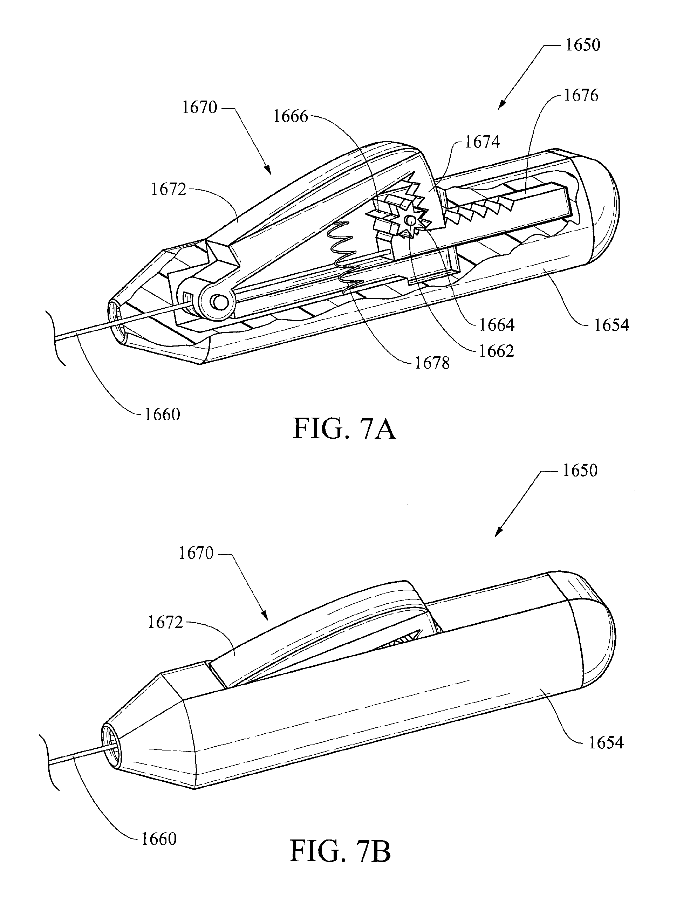

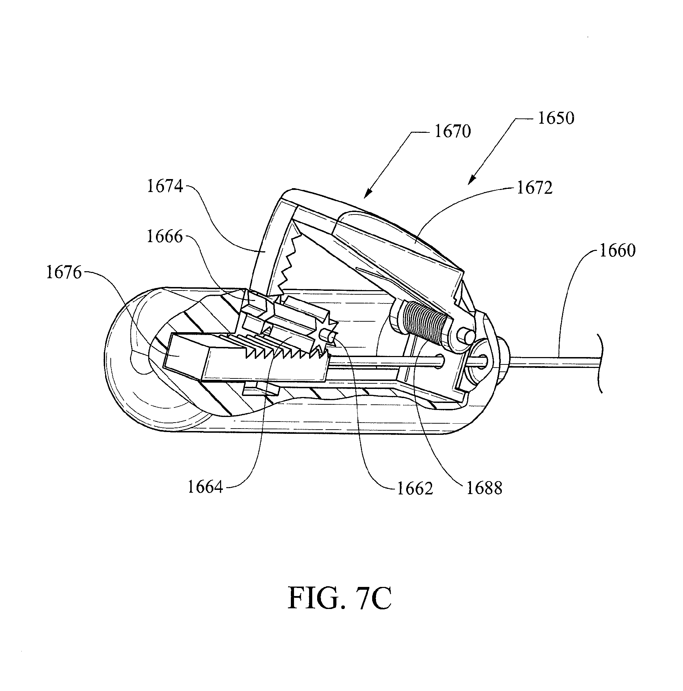

The longer body portion distal of the protuberance can help make certain that, during deployment through a needle, a first fiducial distal of this second fiducial will be fully advanced out of the needle before that second fiducial is positioned for deployment, as will be made clearer with reference to FIGS. 7-8B below. Accordingly, in many preferred embodiments, the fiducial protuberance (of the second and successive fiducials) will be nearer its proximal end than its distal end, so that the distal fiducial body portion projects sufficiently distally that it will advance the preceding first fiducial completely out of the needle lumen by the time that the second fiducial is in a position to be deployed (see FIGS. 4A-4C, 7B, 8B, and corresponding text). It should be appreciated that, even if all surfaces of the central fiducial portion 402 and protuberance 408 are generally smooth, the preferred materials forming the fiducial 400 and the presence of the protuberance 408 may provide a desirable echogenic profile that is readily visualizable under ultrasound at a resolution sufficient for locating and/or navigating it in a patient's body.

The fiducial 400 has a generally cylindrical body 402 formed as a mass with a generally circular transverse cross-section along its proximal and distal end sections. A protuberance 408 projects from the longitudinal circumferential face 406 of the fiducial body 402. As viewed from the top, the protuberance 408 is generally obround. The irregular shape and increased surface area (as compared to a typical cylindrical fiducial of the type used in plug-ended systems and/or systems with some type of lumen-occupying detent) preferably enhances the echogenicity of the fiducial, which preferably will already be desirably high due in part to its composition.

The protuberance 408 includes protuberance end faces 407 that may provide one or more of chamfered, filleted, and radiused transition to the outer face 406 of the body 402. The body 402 is generally a right cylinder, but for the protuberance 408. In this embodiment, the protuberance 408 is rounded and substantially parallel to the longitudinal central axis of the fiducial body, and it is about one half the length of the body 402, and it is centered along the body length. In a preferred embodiment, the fiducial 400 is configured and dimensioned for passage through and release from a needle lumen. For an endoscopic delivery system, the fiducial body (exclusive of the protuberance) will have an outer diameter (OD) of about the same or less than the inner diameter (ID) of a needle lumen, but the fiducial body OD preferably will be no greater than the needle ID. The protuberance 408 will engage and ride along through a needle slot.

Dimensions of one exemplary embodiment are also described with reference to FIGS. 2A-2C. In one exemplary embodiment the body 402 is about 0.12 inches (3.05 mm) long and has an OD of about 0.034 inches (0.86 mm). The protuberance 408 is about 0.06 inches (1.5 mm) long and is aligned along a midline of the body. The protuberance 408 projects about 0.008 inches (0.2 mm) above the OD of the body 402 and is about 0.011 inches (0.28 mm) wide. These measurements and proportions may be varied in other embodiments while remaining within the scope of the presently-claimed material. For example, the protuberance may be more distally or proximally located, and may be at an angle relative to the midline such that it partially spirals around the outer surface of the body.

FIG. 2C shows an end view of a transverse section taken along line 2C-2C of FIG. 2A. It shows one embodiment of general proportions of a fiducial body and protuberance of the present system.

FIG. 3 shows an embodiment of a fiducial introduction needle 800. The needle 800 is illustrated with a beveled distal tip 802. Its tubular cannula body 804 includes a longitudinal needle slot 806 along a distal end region of the cannula 804. The slot 806 preferably includes at least one detent including at least one detent surface, and more preferably two detents. The slot 806 is shown as being open through the entire wall of the cannula 804, but it should be appreciated that the slot may extend less than the thickness of the needle wall, such that it is embodied as a groove.

In the embodiment of FIG. 3, the detent is formed as a narrowed portion 807 of the slot 806 between two tabs 808. The tabs 808 are generally trapezoidal, but may have a different geometry in other embodiments. As shown in FIG. 3A, in certain preferred embodiments, the tabs 808 may be located immediately adjacent the distal bevel (e.g., to maximize efficiency of advancing a fiducial past them and out of the needle while minimizing residual overlap of a deployed fiducial with the beveled portion of the distal needle tip). Each of the transitions between the edge 806a of the needle slot 806, the proximal tab edge 808a, central tab edge 808b, and distal tab edge 808c may be cornered (e.g., chamfered or filleted) or rounded (e.g., radiused). The tabs 808 preferably are near the distal end of the slot 806.

The body wall cannula 804 generally circumferentially defines a needle lumen 810 configured to allow sliding passage therethrough of a fiducial such as, for example, a fiducial (e.g., as shown in FIGS. 2A-2C or others that would readily pass through the needle lumen 810, preferably with controllable retention of the fiducial(s) by the tabs 808). The needle may be constructed from a nickel-titanium alloy, cobalt-chromium (CoCr) alloy, stainless steel or any other suitable material. Its tip may have a different geometry than the beveled configuration shown. In an alternative embodiment, the tabs 808 may meet such that they will be forced to flex upward and/or outward to a greater degree to allow passage of a protuberance on a fiducial. And, the outer surface of the needle may be dimpled or otherwise textured to provide enhanced echogenicity.

An exemplary needle embodiment is also described with reference to FIG. 3, which exemplary needle embodiment may be configured and dimensioned for use with the exemplary fiducial embodiment described above with reference to FIGS. 2A-2C. In one such exemplary needle embodiment, the ID of the needle lumen is at least about 0.034 inches (0.86 mm). The OD of the needle is about 0.042 inches (1.07 mm; about 19-gauge), with a wall-thickness of about 0.008 inches (0.2 mm). The slot portion proximal of the tabs is about 0.02 inches (0.5 mm) wide and about 0.42 inches (about 10.7 mm) long. Each of the tabs extends about 0.06 inches (0.15 mm) out of the slot edge and has a slot-facing edge that is about 0.02 inches (0.5 mm) long (not including the proximal and distal angled transitions from the slot edge, which are radiused at about 0.005 inches (0.13 mm)). These measurements and proportions may be varied in other embodiments, including those illustrated herein, while remaining within the scope of the presently-claimed material. For example, the particular dimensions of a slot, tabs, and fiducial may be configured for use with a 22-gauge needle having a desirable balance of flexibility and stiffness, as well as including a distal needle tip bevel of about 30°, a slot width of about 0.014 inches (about 0.36 mm) with slot tabs separated only by about 0.006 inches (about 0.15 mm) across the slot, and echogenicity-enhancing surface dimpling disposed along the needle exterior adjacent and generally parallel with at least a distal length of the slot.

The distal end portion of a fiducial deployment system 1000 is described with reference to FIG. 4, which is an external view, FIG. 4A which is a longitudinal section view taken along line 4A-4A of FIG. 4, using the needle 800 and fiducial 400 described above, and FIG. 4B, which shows a transverse section view along line 4B-4B of FIG. 4A. The system 1000 includes a flexible elongate needle sheath 1002. The needle 800, including a more flexible proximal body portion 820 extends through a sheath lumen 1004. At least one fiducial 400, illustrated here as a plurality of fiducials 400, is disposed slidably removably in a distal region of the needle lumen 810 of the needle's cannular body. The central longitudinal body portion 402 substantially occupies the inner diameter of the needle lumen 810. The protuberance 408 of each fiducial 400 has a height that may be about the same as the thickness of the needle wall, including the slot 806 into which the protuberances 408 project.

The protuberance 408 of the distal-most fiducial 400 is captured against the tabs 808 of the needle 800. A stylet 1006 configured for use as a pusher is disposed through a portion of the needle lumen 810 and preferably is configured for actuation from the proximal end, whereby it can be used to distally advance/ push out the fiducials and/or hold them in place as the needle is withdrawn from around them. The presence of the fiducials and stylet in the needle 800 preferably improve its columnar strength reduce the likelihood that it will get bent, crimped, or otherwise damaged as it is navigated through and out of the distal end of an endoscope working channel (not shown).

FIG. 4B shows a transverse section end view of a section of a needle 800 (as in FIG. 3) and a fiducial 400 (as in FIGS. 2A-2C). This view shows the preferred close tolerances and a preferred orientation of the fiducial body relative to the needle lumen 810 and the protuberance 408 relative to the needle slot 806.

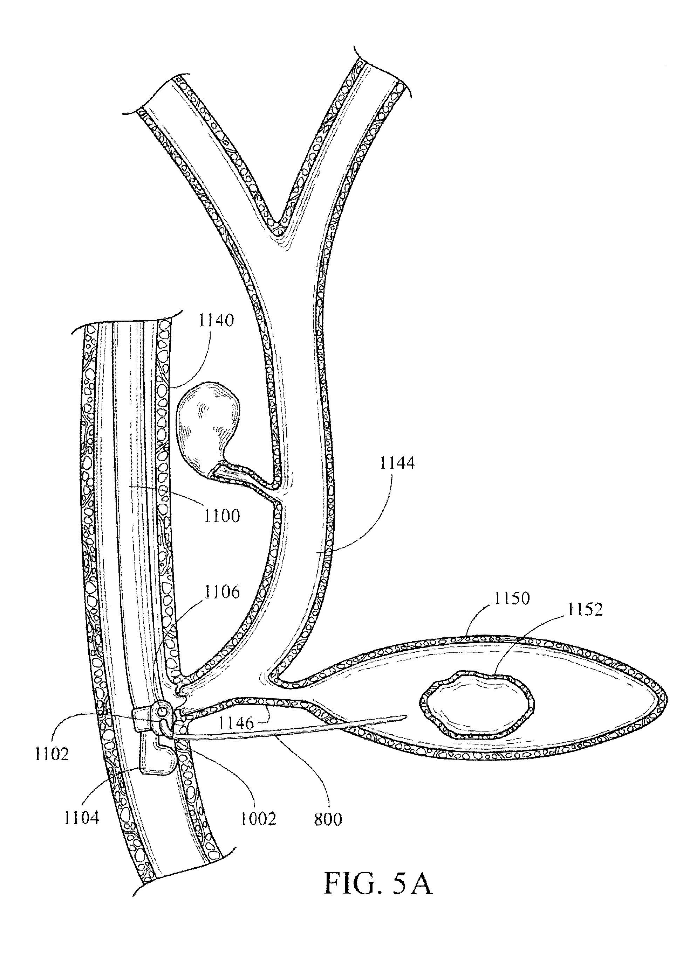

Several different handle embodiments may be used to effect advancement and release of one or more fiducials. Certain handle embodiments are described with reference to FIGS. 7A-8B below, including with reference to the structure and method described below with reference to FIGS. 4-4B and 5A-5C.

A method of using the fiducial deployment needle of FIGS. 4-4B is described with reference to FIGS. 5A-5C, with reference to the structures shown in greater detail in FIGS. 4-4B. In a preferred method of use, an endoscope 1100 is provided, including a working channel 1102. In one preferred method, the endoscope is an EUS endoscope including a distal ultrasound array 1104 configured for ultrasound imaging. The endoscope 1100 preferably also includes a video element 1106 (e.g., CCD, optical camera, or other means for optical visualization). The methods below are described with reference to placing fiducials 400 at the margins of a tumor 1152 of a patient's pancreas 1150, such that the needle body will be of sufficient length and navigability (e.g., pushability and flexibility) to perorally be directed through a patient's gastrointestinal tract to a target site, including doing so via a working channel of an endoscope such as a gastric endoscope, colonoscope, anuscope, or other visualization/procedure-assisting device.

The endoscope 1100 is shown in FIG. 5A as having been directed through a patient's duodenum 1140 until its distal end portion is adjacent the Sphincter of Oddi 1142, which provides access to the common bile duct 1144 from which the pancreatic duct 1146 branches and leads to the pancreas 1150.

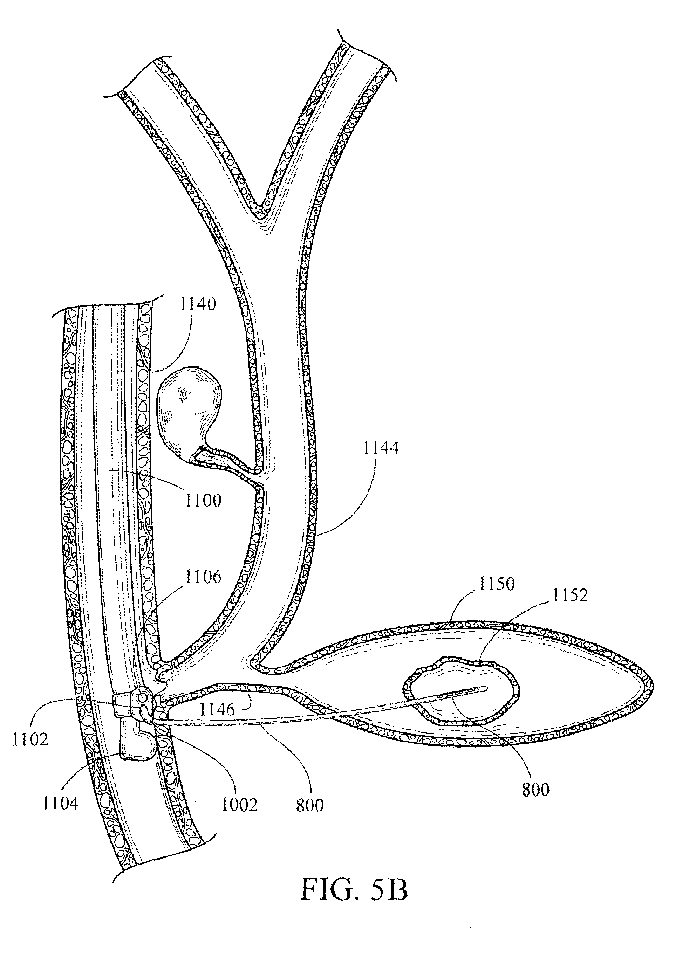

As shown in FIG. 5A, the sheath 1002 has been advanced to the duodenal wall and the needle 800 has been pierced therethrough, extending near the pancreatic duct 1146 to a location adjacent the tumor 1152 in the pancreas 1150. As shown in FIG. 5B, the needle 800 is directed to a first target site at a margin of the tumor 1152 (preferably under ultrasound guidance, which can be replaced, complemented, and/or verified by fluoroscopy or another visualization technique). Once the distal end 802 of the needle 800 is positioned at the first target, the distal-most fiducial 400 therein is deployed. In one aspect, the deployment may be accomplished by positioning the distal needle end 802 and the fiducial 400 therein at the first target, then retracting the needle 800 while retaining the position of the stylet 1006 such that the fiducial 400 remains in the desired first target position. In another aspect, the deployment may be accomplished by positioning the distal needle end 802 and the fiducial 400 therein adjacent the first target, then holding the needle 800 in position while advancing the stylet 1006 such that the fiducial 400 is advanced into the desired first target position.

As will be appreciated from the structure of the needle 800 and fiducials 400 as shown in FIGS. 4-4B, a user preferably will be able to control advancement/ deployment of the fiducials to one at a time, such that a plurality of fiducials (without any spacers) may serially - but separately and independently - directed into different locations. Then the fiducial 400 is in a "ready to deploy" position, its distal protuberance face 408a is engaged against the proximal tab edges 808a. To deploy the fiducial 400, the user must move one of the stylet 1006 or needle 800 relative to the other with sufficient force to advance the protuberance 408 through the tabs 808.

The user preferably will have a tactile sense of resistance as the protuberance 408 passes through the tabs 808, which resistance will decrease immediately as soon as the protuberance clears the tabs. Then the user preferably continues the relative motion of stylet and needle until resistance is again encountered, indicating that the next fiducial behind the distal-most one has met the proximal tab edges 808a.

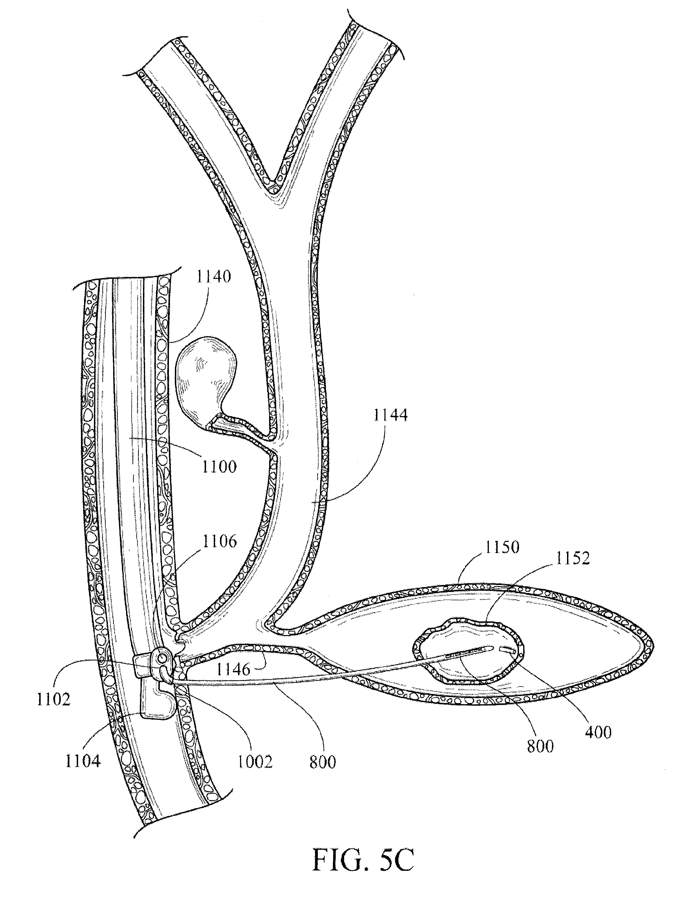

It will often be preferred that the fiducials (and the protuberances thereon) be proportioned such that complete deployment of a distal-most fiducial includes it substantially clearing the distal needle tip 802 and coincides with the protuberance of the next distal-most fiducial meeting the proximal tab edges 808a. As such, it may be advantageous in some fiducial embodiments to position the protuberance more proximally on the fiducial body such that a fiducial body portion distal of the protuberance is longer than a body portion proximal of the protuberance. It should be appreciated that the protuberance of almost any fiducial embodiment in keeping with principles of the present invention may be disposed near the proximal end up to and including flush with the proximal end of the fiducial body). FIG. 5C shows the fiducial in place, with the needle withdrawn away from it.

Next, the user may retract the needle 800 into the sheath 1002 to a sufficient distance allowing it to be re-extended to a second target site, where the procedure described above may be repeated. These steps may be repeated for placement of third, fourth, and further fiducials. As is known in the art, these fiducials may be used for "positive targeting" and/or "negative targeting" of a therapy such as radiation therapy ("positive targeting" indicating "treat here", and "negative targeting" indicating "do not treat here"). The present system presents numerous advantages. For example, consider a patient already undergoing an endoscopy procedure to biopsy a located but undiagnosed tissue mass. The endoscopic biopsy can be taken and a tissue slide prepared immediately. If a diagnosis is made (in conjunction with whatever other data are available and pertinent) that the tissue mass will benefit from a treatment where placement of fiducials is indicated, the physician can immediately deploy fiducials in the manner described above.

The ability to complete the method using direct/ video and ultrasound imaging with little or no use of fluoroscopy presents an advantage of minimizing the radiation exposure of the patient (who may, for example, have to undergo radiation therapies where the total amount of exposure to radiation is desired to be minimized to that which is therapeutically and diagnostically necessary). Advantages of time and expense for the patient, physician and other treating/diagnostic personnel, and the treatment facility are likely as implementation of the present method may prevent all of those entities from having to schedule and conduct a second endoscopic procedure, and/or to extend the initial diagnostic procedure with the time-consuming methods and materials currently available in the prior art as described. It should also be appreciated that, when informed by the present disclosure, those of skill in the art may utilize and/or adapt the presently-disclosed embodiments for percutaneous use while remaining within the scope of one or more claims.

Fiducials with generally cylindrical or otherwise generally regular geometry may migrate after having been placed in a desired location, including that - over the course of multiple treatments of a target area delineated by fiducials - they may migrate with changes in the condition of surrounding tissues. For circumstances where it may be advantageous to minimize migration, a fiducial may be used that includes one or more anchoring projections.

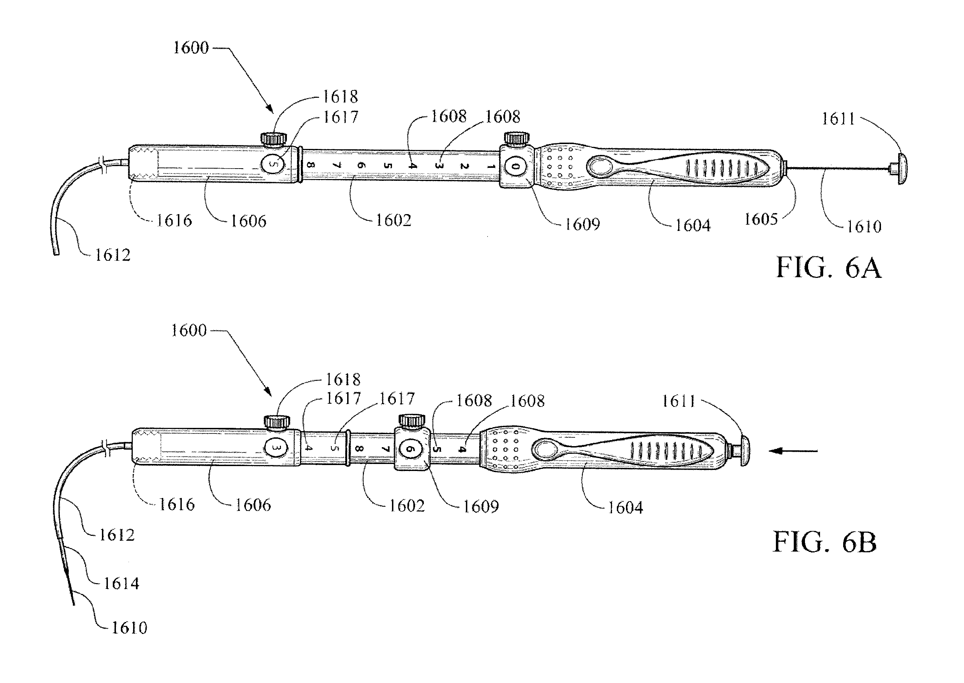

FIGS. 6A-6B show a handle embodiment 1600 that may be used with a fiducial deployment system. The handle 1600 includes a sheath-attached handle member 1602 with a needle-attached handle member 1604 longitudinally slidably disposed on its proximal end. A handle member 1606 (which may be configured for scope-attachment) is slidably attached to the distal end of the sheath-attached handle member 1602. The sheath-attached handle member 1602 is attached to the needle sheath 1612 and the needle-attached handle member 1604 is attached to the needle 1614 (which may be configured in the manner of any of the needles disclosed herein or later developed in accordance with principles of the present disclosure). The scope-attachment handle member 1606 is configured for incrementally fixable, longitudinally-adjustable (relative to the other handle components) attachment to the exterior of an endoscope working channel (not shown) using, for example, a threaded cavity 1616. The scope-attachment handle member 1606 allows a user to determine the distance by which the sheath 1612 will extend from a standard-length endoscope, and it may include numerical or other indicia 1617 corresponding to that relative length and an adjustable engagement structure 1618 allowing a user to select a length and engage the scope-attachment handle member 1606 accordingly. It should be appreciated that embodiments of the handle described and claimed herein may be practiced within the scope of the present invention without including a scope-attachment member.

The sheath-attached handle member 1602 includes numerical indicia 1608 and an adjustable ring 1609 that limits the movement of the needle-attached handle member 1604 and provides a way to select the distance to which the needle 1614 may be extended beyond the sheath 1612. By way of illustration, the configuration shown in FIG. 6A would allow the sheath to extend 5 units (e.g., inches, cm) beyond the distal end opening of an endoscope working channel, and the needle 1614 would not extend at all beyond the distal end of the sheath 1612. The configuration shown in FIG. 6A would allow the sheath to extend 3 units (e.g., inches, cm) beyond the distal end opening of an endoscope working channel, and the needle 1614 would be allowed to extend up to 6 units beyond the distal end of the sheath 1612, although its current position would be only about 4 units beyond the distal end of the sheath 1612.

A stylet 1610 extends through a lumen of the needle 1614 and has a stylet cap 1611 fixed on its proximal end. The stylet 1610 is shown as being retracted proximally in FIG. 6A, and extended beyond the distal end of the needle 1614 in FIG. 6B. The stylet 1610 may be manually advanced distally through the needle lumen in the same manner as described above (with reference to FIGS. 4-4B) for a stylet 1006. As such, a user may use the stylet to manually push fiducials out of a distal end of the needle 1614. If this method is used (e.g., in the manner described above for deployment of fiducials with reference to FIGS. 4-5C), a user may rely upon tactile feedback to determine when a fiducial has been advanced beyond any detents, which may be difficult through a long stylet - particularly if the detents are rounded such that the advancing motion is relatively smooth. Accordingly, it may be advantageous to provide an advancement mechanism configured to attach to (including being integrated with) the handle 1600 that provides improved control of stylet advancement.

FIGS. 7A-8B show embodiments of advancement mechanisms that may be used with the handle assembly configurations of FIGS. 6A-6B, or other handle configurations (including, for example, those disclosed in U.S. Pat. App. Publ. Nos. 2010/0280367 and 2011/0152611 to Ducharme et al., U.S. Pat. App. Ser. Nos. 13/526,991 to McHugo et al., 13/526,008 to Lavelle et al., and 13/645,614 to Murray et al.). FIGS. 7A-7B and 7C show a clutched-gear handle component 1650 for a fiducial deployment system. In certain embodiments, the clutched gear component 1650 may be removably or permanently attached to a proximal end 1605 of a handle such as the one shown in FIGS. 6A-6B, where it will provide means for controlled advancement of a stylet (e.g., stylet 1610) in lieu of direct and/or manual manipulation of the stylet cap 1611.

The clutched gear handle component 1650 may include an actuation member 1670 and an elongate first handle member 1654 including and defining a central longitudinal axis. It may be attached to an elongate distal outer body having a longitudinal body lumen (e.g., in some embodiments, needle-attached handle member 1604, or - in other embodiments - a fiducial needle and/or sheath). A stylet 1660 (which may correspond to the stylet 1610) extends through at least a portion of the first handle member 1654 along or generally aligned with its central longitudinal axis.

An axle 1662 is mounted rotatably within the first handle member 1654, disposed adjacent to and transverse of the central longitudinal axis. A clutched gear 1664 is mounted rotatably to the axle 1662 by a one-way clutch bearing allowing unidirectional rotation of the clutched gear 1664 around the axle 1662. A drive gear 1666 is fixed to the axle 1662, such that the drive gear rotates with the axle, and that rotation can be unidirectionally actuated by rotation of the clutched gear 1664 in a first direction, while counter-rotation of the clutched gear in the opposite direction will not rotate the axle and drive gear. Those of skill in the art should appreciate that a ratchet/pawl mechanism may be adapted for use as a function equivalent of the clutched gear, within the scope of the present invention.

An actuation member, embodied in FIGS. 7A-7C as a generally L-shaped lever 1670, may be provided. It includes a first arm 1672 generally longitudinally aligned with the first handle member 1654 and attached reciprocatingly pivotably to the first handle member 1654 along an axis transverse to the central longitudinal axis thereof and transverse to a longitudinal axis of the lever 1670. A second arm 1674 of the lever 1670 extends toward the central longitudinal axis and is in mechanical communication with one of the drive gear 1666 or the clutched gear 1664. A spring, such as a coil spring 1678 in FIG. 7A or a torsion spring 1688 in FIG. 7C, may be provided to facilitate reciprocating actuation of the lever 1670. As used here, the term "generally L-shaped" includes "V-shaped" and is used as a term of convenience encompassing generally a member having at least two arms angled about 90 degrees plus/minus about 45 degrees relative to each other.

A rack member 1676 is engaged and generally longitudinally aligned with the stylet 1660. A surface of the rack member 1676 mechanically communicates with a surface of the other of the drive gear 1666 or the clutched gear 1664 such that actuation of the actuation member 1670 is mechanically translated to unidirectional movement of the stylet 1660 via the rack and gears. The L-shaped lever 1670, the rack 1676, the drive gear 1666, and the clutched gear 1664 are shown in this embodiment as including at least one toothed surface, and the mechanical communication is provided by interengagement of at least two of the toothed surfaces. Other forms of frictional or mechanical engagement may be used to provide the mechanical communication.

The mechanical communication of the actuation mechanism 1650 may be configured such that a predetermined number of actuations of the lever 1670 will advance the stylet 1660 a lengthwise distance corresponding to dispensing a single fiducial 400 out of a distal end of a needle 1614. This is indicated in FIG. 7B, which shows a distal needle 1614 with a stylet 1660 having been advanced by actuation of the lever 1670 to dispense a single fiducial 400 (e.g., via intervening structure such as the fiducial deployment device embodiment shown in FIG. 6A, with the clutched gear component 1650 attached at end 1605 of the handle element 1604).

Those of skill in the art will appreciate that actuation may user-perceptibly be indexed by visual indicia, tactile indicia, audible indicia, or any combination thereof, and that the indicia may be configured to correspond to a pre-determined longitudinal movement distance of the stylet by the rack(s). A variety of such indicia are known and well within the skill in the art, given the present disclosure. For example one of the rack elements may include numerals, colored bands, or other visual indicia made viewable through a side window in the handle body (e.g., 1658, 1684, 1984). Types of indicia may include those disclosed in U.S. Pat. No. 6,613,002, which is incorporated by reference herein (except, as those of skill in the art will appreciate, placed proximally on the stylet, one or more racks, and/or other components such that a user may easily observe the handle embodiment and determine the number of fiducials dispensed and/or the distance of stylet advancement). A numeric or other "counter" type of indicator may be provided, attached to an actuator element, the stylet, or other component and configured to indicate the number of fiducials that have been dispensed and/or the number of fiducials remaining. This includes those described in U.S. Pat. App. Publ. 2011/0152611, which is incorporated herein by reference in its entirety. Opposed detents on a static component and one or more moving elements (e.g., rack, stylet) may easily be included to provide tactile and/or auditory feedback in addition to providing information about the distance of stylet advancement and/or needle retraction associated with a particular actuation movement. A method of use may include actuation of the lever 1670 to advance the stylet 1660 for deployment of one or more fiducials in one or more locations. Because deployment of multiple fiducials in a single location/actuation is disfavored (due, for example, to "train-wreck" misalignment and a desire to minimize use of fiducial materials), preferred method embodiments will deploy only one fiducial at a time.

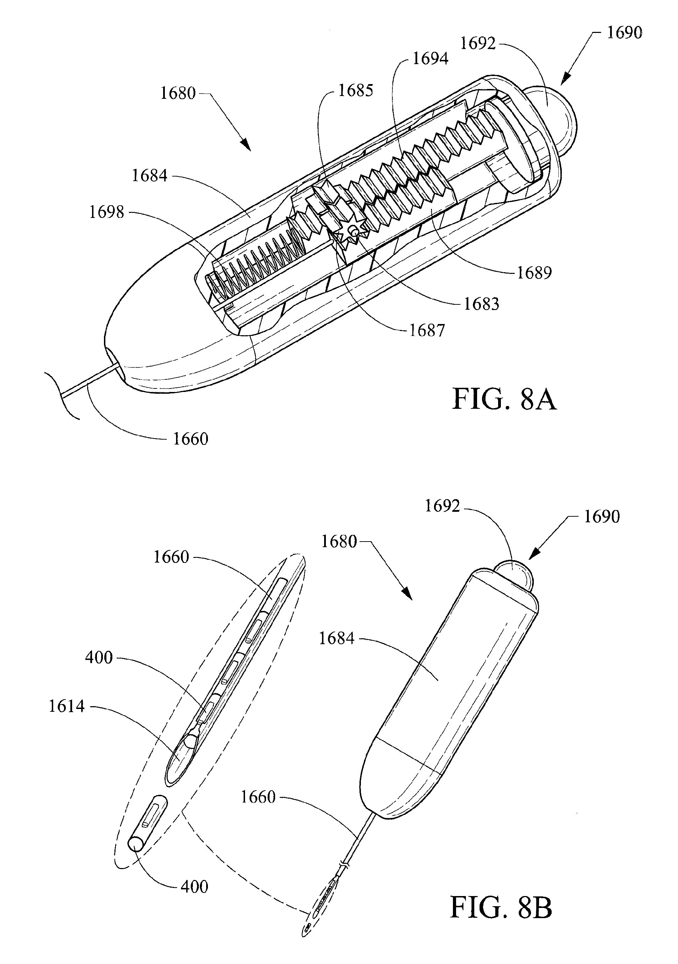

FIGS. 8A-8B show another embodiment of a clutched-gear handle advancement mechanism that may be used with the handle assembly configurations of FIGS. 6A-6B, or other handle configurations (including, for example, those disclosed in U.S. Pat. App. Publ. Nos. 2010/0280367 and 2011/0152611 to Ducharme et al., U.S. Pat. App. Ser. Nos. 13/526,991 to McHugo et al., 13/526,008 to Lavelle et al., and 13/645,614 to Murray et al.). FIGS. 8A-8B show a clutched-gear handle component 1680 for a fiducial deployment system. In certain embodiments, the clutched gear component 1680 may be removably or permanently attached to a proximal end of a handle such as the one shown in FIGS. 6A-6B, where it will provide means for controlled advancement of a stylet (e.g., like stylet 1610) in lieu of the stylet cap 1611.

The clutched gear component 1680 may include an actuation member 1690 and an elongate first handle member 1684 including and defining a central longitudinal axis. It may be attached to an elongate distal outer body having a longitudinal body lumen (e.g., in some embodiments, needle-attached handle member 1604, or - in other embodiments - a fiducial needle and/or sheath). A stylet 1660 (which may correspond to the stylet 1610) extends through at least a portion of the first handle member 1684 along or generally aligned with its central longitudinal axis.

An axle 1683 is mounted rotatably within the first handle member 1684, disposed adjacent to and transverse of the central longitudinal axis. A clutched gear 1685 is mounted rotatably to the axle 1683 by a one-way clutch bearing allowing unidirectional rotation of the clutched gear 1685 around the axle 1683. A drive gear 1687 is fixed to the axle 1683, such that the drive gear rotates with the axle, and that rotation can be unidirectionally actuated by rotation of the clutched gear 1685 in a first direction, while counter-rotation of the clutched gear in the opposite direction will not rotate the axle and drive gear.

An actuation member, embodied in FIGS. 8A-8B as a reciprocating proximal-end button 1692, may be provided. It includes a first rack 1694 generally longitudinally aligned in the first handle member 1684. The first rack 1694 is in mechanical communication with one of the drive gear 1687 or the clutched gear 1685. A spring, such as a coil spring 1698, may be provided to facilitate reciprocating actuation of the button 1692.

A second rack member 1689 is engaged and generally longitudinally aligned with the stylet 1660. A surface of the second rack member 1689 mechanically communicates with a surface of the other of the drive gear 1687 or the clutched gear 1685 such that actuation of the actuation member 1690 (e.g., depressing the button 1692) is mechanically translated to unidirectional movement of the stylet 1660 via the racks and gears. The first rack 1694, the second rack 1689, the drive gear 1687, and the clutched gear 1685 are shown in this embodiment as each including at least one toothed surface, and the mechanical communication is provided by interengagement of at least two of the toothed surfaces. Other forms of frictional or mechanical engagement may be used to provide the mechanical communication.

The mechanical communication of the actuation mechanism 1690 may be configured such that a predetermined number of actuations of the button 1692 will advance the stylet 1660 a lengthwise distance corresponding to dispensing a single fiducial 400 out of a distal end of a needle 1614. This is indicated in FIG. 8B, which shows a distal needle 1614 with a stylet 1660 having been advanced by actuation of the button 1692 to dispense a single fiducial 400 (e.g., via intervening structure such as the fiducial deployment device embodiment shown in FIG. 6A, with the clutched gear component 1680 attached at end 1605 of the handle element 1604). Those of skill in the art will appreciate that actuation may user-perceptibly be indexed by visual indicia, tactile indicia, audible indicia, or any combination thereof, and that the indicia may be configured to correspond to a pre-determined longitudinal movement distance of the stylet by the rack(s). A variety of such indicia are known and well within the skill in the art, given the present disclosure.

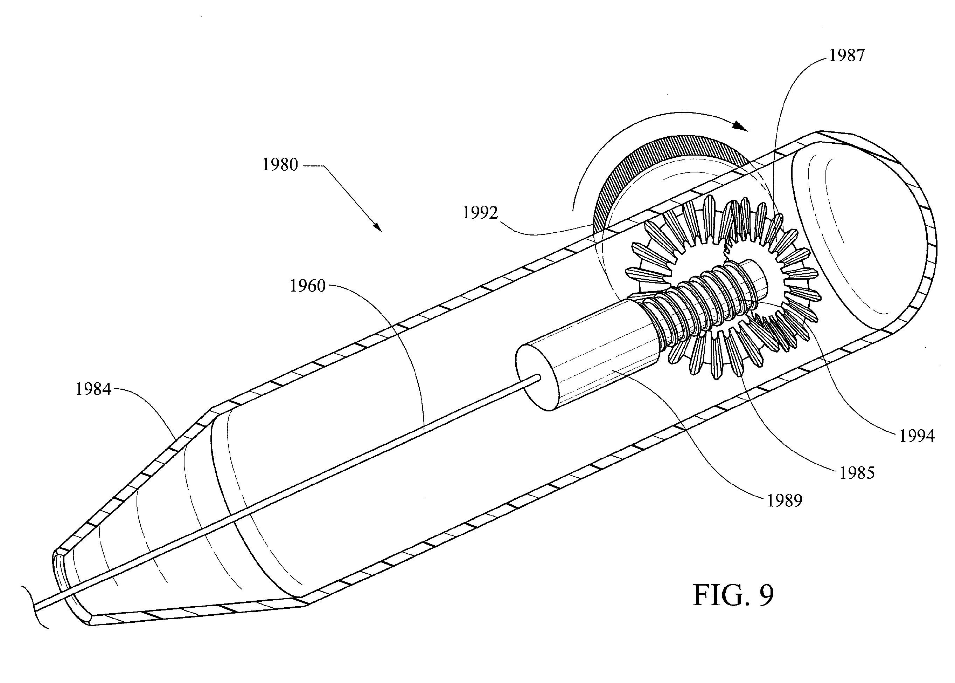

FIG. 9 shows another embodiment of a clutched-gear handle advancement mechanism that may be used with the handle assembly configurations of FIGS. 6A-6B, or other handle configurations (including, for example, those disclosed in U.S. Pat. App. Publ. Nos. 2010/0280367 and 2011/0152611 to Ducharme et al., U.S. Pat. App. Ser. Nos. 13/526,991 to McHugo et al., 13/526,008 to Lavelle et al., and 13/645,614 to Murray et al.). FIG. 9 shows a longitudinal longitudinal-cutaway view of a clutched-gear handle component 1980 for a fiducial deployment system. In certain embodiments, the clutched gear component 1980 may be removably or permanently attached to a proximal end of a handle such as the one shown in FIGS. 6A-6B, where it will provide means for controlled advancement of a stylet 1960 (e.g., like stylet 1610) in lieu of the manually manipulable stylet cap 1611.

The clutched gear component 1980 may include a rotary actuation member 1992 and an elongate first handle member 1984 (the wall/housing of which is shown as longitudinally sectioned to expose the internal components) including and defining a central longitudinal axis. It may be attached to an elongate distal outer body having a longitudinal body lumen (e.g., in some embodiments, needle-attached handle member 1604, or - in other embodiments - a fiducial needle and/or sheath). The stylet 1960 (which may correspond to the stylet 1610) extends through at least a portion of the first handle member 1984 along or generally aligned with its central longitudinal axis.

An actuation member, embodied in FIG. 9 as a rotating knob 1992, may be provided. It is mounted to an axle (not shown), which is mounted rotatably through the wall of the first handle member 1984, transverse of the central longitudinal axis. A clutched gear 1985 is mounted rotatably to the axle by a one-way clutch bearing allowing unidirectional rotation of the clutched gear 1985 around the axle when the knob 1992 is rotated. Those of skill in the art will appreciate that the unidirectionally-rotatable clutch-axle interface may be on the gear-end and/or on the knob-end of the axle. A drive gear 1987 meshes or otherwise mechanically communicates with the clutched gear 1985, such that the drive gear rotation can be unidirectionally actuated by rotation of the knob 1992 and the clutched gear 1985 in a first direction, while counter-rotation of the knob 1992 in the opposite direction will not generate rotation of the clutched gear 1985 or the drive gear 1987.

The drive gear 1987, shown in FIG. 9 as a beveled gear interfacing with the clutched gear 1985, rotates around the longitudinal axis and is fixed to and includes a first rack 1994 (configured as a threaded cylinder) that is generally longitudinally aligned in the first handle member 1984. A second rack member 1989 is threadedly engaged into and generally longitudinally aligned with the first rack 1994 and is engaged and generally longitudinally aligned with the stylet 1960 at the opposite end (of the second rack). As such the internal threaded surface of the second rack member 1989 mechanically communicates with the outer threaded surface of the drive gear 1987 such that actuation of the actuation member 1992 (i.e., rotating the knob 1992) is mechanically translated to unidirectional movement of the stylet 1960 via the gears and racks. It should be appreciated that the clutch element described herein may be located anywhere along the path of mechanical communication from the actuation member (e.g. knob 1992) to the stylet, such that the stylet will only move in one direction upon proper actuation of the actuation member (e.g., rotation rather than counter-rotation of the knob 1992), but will not be actuated in reverse by other manipulation of the actuation member.

In certain preferred embodiments, a predetermined incremental rotation of the knob 1992 will advance the stylet a distal longitudinal distance corresponding to the length of (and thereby the dispensing of) one fiducial or another predetermined number of fiducials. Those of skill in the art will appreciate that the predetermined incremental rotation may be one full turn, a partial turn (e.g., with visual, tactile, and/or auditory indicia such as "clicks"), or some other user-controllable increment that advances the stylet 1960 a desired distance. Those of skill in the art will appreciate that other forms of frictional or mechanical engagement may be used to provide the mechanical communication in addition to and/or other than the meshed-gear and threaded interface shown in FIG. 9.

Those of skill in the art will appreciate with reference to the embodiments disclosed above that a predetermined number of fiducials may be released into a desired location by a single actuation of the lever, button, or other actuation member. The predetermined number preferably will be one, but may include a plurality of fiducials. The configuration of the present embodiments provide clear advantages over prior designs that utilize releasable end-plugs in a needle to retain fiducials, and/or that use less refined means of controlling the fiducial release than the notch/tab needle design and/or actuation handles described herein. Drawings and particular features in the figures illustrating various embodiments are not necessarily to scale. Some drawings may have certain details magnified for emphasis, and any different numbers or proportions of parts should not be read as limiting, unless so-designated by one or more claims. Those of skill in the art will appreciate that embodiments not expressly illustrated herein may be practiced within the scope of the present invention, including that features described herein for different embodiments may be combined with each other and/or with currently-known or future-developed technologies while remaining within the scope of the claims presented here. For example, a needle and fiducials of the present system may be used percutaneously, including in another minimally invasive surgical procedure, such as a laparoscopic-type procedure, within the scope of the claimed invention. For example, a target site may be a location in or near the gastrointestinal tract (e.g., liver, pancreas) such as those locations that may be accessible by endoscopy (using a minimally invasive endoscope introduced through a natural patient orifice, e.g., mouth, anus, vagina). This includes -more broadly- sites reachable through NOTES (natural orifice translumenal endoscopic surgery) procedures. The present method and device may also be used with other minimally-invasive surgical techniques such as percutaneous endoscopic procedures (e.g., laparoscopic procedures) or percutaneous non-endoscopic procedures, but most preferably is used with less invasive endoscopy procedures. It is therefore intended that the foregoing detailed description be regarded as illustrative rather than limiting. And, it should be understood that the following claims, including all equivalents, are intended to define the spirit and scope of this invention.

## Claims

1. A clutched-gear handle for a fiducial deployment system including an advancement mechanism comprising:
an actuation member and an elongate first handle member, including a central longitudinal axis defined by an elongate outer body of the first handle member, the outer body including a body lumen longitudinally disposed therethrough;
where a stylet extends through at least a portion of the first handle member along or generally aligned with the central longitudinal axis;
an axle mounted rotatably in the first handle member, disposed adjacent to and transverse of the central longitudinal axis thereof;
a clutched gear mounted rotatably to the axle by a one-way clutch bearing allowing unidirectional rotation of the clutched gear around the axle, and a drive gear fixed to the axle;
where the actuation member is disposed in reciprocating mechanical communication with one of the drive gear or the clutched gear; and
a rack member engaged with and generally longitudinally aligned with the stylet, where a surface of the rack member mechanically communicates with a surface of the other of the drive gear or the clutched gear such that actuation of the actuation member is mechanically translated to unidirectional movement of the stylet via the rack and gears.

2. The clutched-gear handle of claim 1, further comprising a fiducial deployment assembly attached to an elongate needle disposed longitudinally slidably through a sheath lumen of the assembly, wherein the needle includes a needle lumen disposed longitudinally therethrough;
the stylet disposed longitudinally slidably through the needle lumen; and
a portion of the clutched-gear handle configured to attach to a proximal portion of the fiducial deployment assembly in a manner configured for effecting longitudinal movement of the stylet relative to the needle by predetermined increments.

3. The clutched-gear handle of claim 1 or claim 2, where the actuation member is configured as a generally L-shaped lever including a first arm generally longitudinally aligned with the first handle member and attached reciprocatingly pivotably to the first handle member along an axis transverse to the central longitudinal axis thereof and transverse to a longitudinal axis of the lever, where a second arm of the lever extends toward the central longitudinal axis and is in mechanical communication with one of the drive gear or the clutched gear.

4. The clutched-gear handle of claim 3, where at least two of:
a shorter arm of the generally L-shaped lever,
the rack, the drive gear, and
the clutched gear
each includes at least one toothed surface, and
where the mechanical communication is provided by interengagement of toothed surfaces.

5. The clutched-gear handle of any one of the preceding claims, wherein the actuation member is disposed in mechanical communication with a spring that biases the actuation member to a non-actuated position.

6. The clutched-gear handle of any one of the preceding claims, wherein actuation is indexed by visual indicia, tactile indicia, audible indicia, or any combination thereof, and wherein said indicia is configured to correspond to a pre-determined longitudinal movement distance of the stylet by the rack member.

7. The clutched-gear handle of claim 6, where the indicia each correspond to a stylet distance moved sufficient to advance one fiducial or a predetermined plurality of fiducial out of a needle attached to the clutched-gear handle, the needle including a distal end opening.

8. The clutched-gear handle of claim 7, wherein the actuation is indexed by visual indicia, tactile indicia, audible indicia, or a combination thereof, and wherein said indicia of rotation is configured to correspond to a known longitudinal movement distance of the stylet.

9. The clutched-gear handle of claim 7, configured such that moving the actuation member in one direction will advance the stylet distally by a predetermined first distance that will advance at least one fiducial past a detent structure of the needle and out of the distal needle end opening.

10. The clutched-gear handle of any one of the preceding claims, wherein the actuation member is configured as a button aligned with, and communicating mechanically with, the rack via the clutched gear and drive gear.

11. The clutched-gear handle of any one of the preceding claims, further comprising an elongate needle and including a longitudinal needle lumen through which the stylet extends, and where a distal portion of the needle lumen contains a plurality of detent-retained fiducials, deployable in controlled, one at a time, serial fashion by distal advancement of the stylet.

12. The clutched-gear handle of claim 11, where the needle lumen is defined by an inner diameter devoid of detents that would impede distalward progress of a fiducial through the needle lumen, and where the detents are disposed within boundaries defined by the needle wall.

13. The clutched-gear handle of claim 11 or claim 12, further comprising at least one fiducial,
wherein the needle includes
a tubular cannula body defining the needle lumen and
a distal needle end region, the distal end region comprising
a distal needle end opening at a distal end of the needle lumen; and
at least one generally longitudinal needle slot extending radially through at the cannula body and open to the needle lumen, where the needle slot includes at least one detent that does not impede any portion of the needle lumen;
wherein the at least one fiducial comprises
a generally columnar body including
a central fiducial portion slidably disposed in the needle lumen; and
at least one side protuberance projecting into the needle slot; and
wherein the stylet is configured to advance the at least one fiducial past the detent and out of the distal needle end opening; and
optionally further comprising visual indicia of a distance advanced distally by the stylet or of a number of one or more fiducials advanced past the detent and out of the distal needle end opening.

14. The clutched-gear handle of claim 13, where the actuation member is configured as
a generally L-shaped lever including a first arm generally longitudinally aligned with the first handle member and attached reciprocatingly pivotably to the first handle member along an axis transverse to the central longitudinal axis thereof and transverse to a longitudinal axis of the lever, where a second arm of the lever extends toward the central longitudinal axis and is in mechanical communication with one of the drive gear or the clutched gear; or
a button aligned with and attached to the rack.

15. A fiducial deployment system comprising:
a flexible elongate needle sheath configured for passage through a working channel of an endoscope, the needle sheath including
a sheath lumen extending longitudinally through its length;
a flexible elongate needle extending slidably through the sheath lumen, the needle including
a tubular cannula body defining a needle lumen disposed through at least a lengthwise portion of the cannula body and
a distal needle end region, the distal end region comprising
a distal needle end opening at a distal end of the needle lumen; and
at least one generally longitudinal needle slot extending radially through at least a thickness portion of the cannula body and open to the needle lumen, where the needle slot includes at least one detent that does not impede the needle lumen;
a plurality of fiducials each including
a generally columnar body including
a central fiducial portion slidably disposed in the needle lumen; and
at least one side protuberance projecting into the needle slot;
a flexible elongate stylet extending through a portion of the needle lumen and configured to pushingly advance the at least one fiducial past the detent and out of the distal needle end opening; and
a handle including
an actuation mechanism, said actuation mechanism including at least one rotatable gear mechanically communicating between an actuation member, embodied as a lever, a knob, or a button, and at least one rack attached to the stylet, where the mechanism is constructed such that actuating the actuation mechanism rotates the at least one unidirectionally rotatable gear and thereby communicates mechanical force through the at least one rack to the stylet in a manner that distally advances at least one of the plurality of fiducials past the detent and distally out of the needle lumen.
